(19) Europäisches Patentamt
European Patent Office
Office européen des brevets

(11) **EP 3 637 124 A1**

(12) **EUROPÄISCHE PATENTANMELDUNG**

(43) Veröffentlichungstag:
**15.04.2020 Patentblatt 2020/16**

(51) Int Cl.:
***G01R 33/28*** *(2006.01)*

(21) Anmeldenummer: **18199924.4**

(22) Anmeldetag: **11.10.2018**

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**
Benannte Validierungsstaaten:
**KH MA MD TN**

(71) Anmelder: **Siemens Healthcare GmbH**
**91052 Erlangen (DE)**

(72) Erfinder: **Schnetter, Volker**
**90425 Nürnberg (DE)**

Bemerkungen:
Geänderte Patentansprüche gemäss Regel 137(2) EPÜ.

(54) **VERFAHREN ZUR STEUERUNG EINES HOCHFREQUENZVERSTÄRKERS EINER MAGNETRESONANZANLAGE**

(57) Die vorliegende Erfindung betrifft ein Verfahren zur Steuerung eines Hochfrequenzverstärkers (10) einer Magnetresonanzanlage (1). Bei dem Verfahren wird eine von dem Hochfrequenzverstärker (10) abzugebenden Hochfrequenzleistung während einer Untersuchung eines Patienten (4) in der Magnetresonanzanlage (1) bestimmt. Unter Verwendung einer Lambertschen Funktion wird eine Maximaltemperatur im Gewebe des Patienten (4) in Abhängigkeit von der Hochfrequenzleistung bestimmt. Der Hochfrequenzverstärker (10) wird in Abhängigkeit von der Maximaltemperatur eingestellt.

FIG 13

EP 3 637 124 A1

**Beschreibung**

Die vorliegende Erfindung betrifft ein Verfahren zur Steuerung eines Hochfrequenzverstärkers einer Magnetresonanzanlage und eine entsprechend ausgestaltete Magnetresonanzanlage, welche eine schädliche Belastung eines Patienten durch elektromagnetische Strahlen verhindern.

Magnetresonanzuntersuchungen können in der medizinischen Diagnostik verwendet werden. Ein Vorteil der Magnetresonanzuntersuchung liegt darin, dass ein Patient bei der Untersuchung keiner radioaktiven oder ionisierenden Strahlung ausgesetzt wird, wie beispielsweise bei einer Röntgenuntersuchung. Dennoch wird der Patient bei einer Magnetresonanzuntersuchung elektromagnetischen Feldern ausgesetzt, welche von dem Gewebe des Patienten absorbiert werden können. Zur Erzeugung von Magnetresonanzbildern wird während einer Magnetresonanzuntersuchung ein rotierendes Magnetfeld (B-Feld) erzeugt, welches zu einer induzierten Spannung führt, wodurch Ströme im Patienten fließen können. Diese induzierten Ströme und gegebenenfalls weitere Verschiebeströme können zu einer Erwärmung des Gewebes des Patienten führen. Diese Erwärmung ist bei der Durchführung einer Magnetresonanzuntersuchung zu begrenzen. Als Maß für die Absorption von elektromagnetischer Feldenergie in biologischem Gewebe wird die sogenannte spezifische Absorptionsrate (SAR) verwendet. In den Druckschriften US 6762605 B2, US 9625541 B2, US 10031193 B2, US 9989603 B2, US 8547097 B2 und US 9547053 B2 werden verschiedene Verfahren auf dem Gebiet der SAR-Kontrolle beschrieben.

Zur Gewährleistung des Schutzes des Patienten vor einer unzulässig hohen Belastung mit elektromagnetischen Feldern und vor einer unzulässig starken Erwärmung des Gewebes während einer Magnetresonanzuntersuchung wird beispielsweise gemäß Normen (zum Beispiel IEC 60601-2-33) die SAR begrenzt. Dabei wird beispielsweise davon ausgegangen, dass eine Ganzkörper-SAR von 4 W/kg die Körperstammtemperatur um maximal 1° C erhöht. Bei Sendespulen, die kein homogenes B1-Feld zur Bildgebung erzeugen, muss die lokale SAR begrenzt werden. Hierfür können ebenfalls entsprechende Grenzwerte festgelegt sein. Neuere Simulationsrechnungen haben gezeigt, dass auch bei einem homogenen B1-Feld die lokale SAR über dem in der Norm festgelegten Wert liegen kann, ohne das Gewebe des Patienten zu schädigen, d.h. es können höhere Strahlungsintensitäten verwendet werden, ohne den Patienten zu schädigen.

Daher kann es wünschenswert sein, die Gewebeschädigung durch Wärme exakter zu bestimmen. Ein möglicher Ansatz dafür ist die Berechnung des CEM43-Wertes (CEM43: Cumulative Equivalent minutes at 43 Degrees C), wie es beispielsweise in der Veröffentlichung "Virtual Population-Based Assessment of the Impact of 3 Tesla Radiofrequency Shimming and Thermoregulation on Safety and B1+ Uniformity", M. Murbach, et al., Magnetic Resonance in Medicine 76:986-997 (2016) beschrieben wird. Des Weiteren wird dies im Rahmen der Gruppe IEC TC62 SC62B MT40 (Magnetic resonance equipment for medical diagnosis) und in ISO14708-3:2017(E): Implants for surgery - Active implantable medical devices - part 3: Implantable neurostimulators, mit gewebespezifischen CEM43-Grenzen diskutiert.

Als Ausgangspunkt kann beispielsweise folgende abgeleitete Definitionsgleichung aus dem Schädigungsintegral von Arrhenius verwendet werden:

$$CEM43(\tau) = \int_0^\tau R^{T_{start}+T(t)-43°C}dt \qquad (1)$$

In der oben genannten Veröffentlichung (Murbach et al.) sowie in E. Neufeld, et al., "Rapid method for thermal dose-based safety supervision during MR scans", Biomagnetics 2015:10.1002/bem.21919 wird ausgehend von diesem Schädigungsintegral davon ausgegangen, dass die Temperaturerhöhung im Gewebe im Wesentlichen einer Wurzelfunktion folgt, wenn ein Perfusionsmodell mit exponentieller Perfusionssteigerung angenommen wird. Dies stellt jedoch nur eine Näherung dar, wodurch eine unnötige Begrenzung der elektromagnetischen Felder bedingt werden kann. Ferner kann es erforderlich sein, die elektromagnetischen Felder weiter zu begrenzen, um trotz der Näherung eine Gewebeschädigung zuverlässig zu verhindern.

Die Aufgabe der vorliegenden Erfindung ist daher, die sich einstellende Temperatur im Gewebe infolge einer Magnetresonanzuntersuchung möglichst exakt zu bestimmen, um darauf aufbauend eine effiziente CEM43-Überwachung zu realisieren. Diese Überwachung kann vor dem Erreichen eines Grenzwertes abschalten, um eine Schädigung im Gewebe des Patienten zu verhindern. Dabei kommt es nicht auf die exakte räumliche Temperaturverteilung an, sondern auf eine praktische Bewertung der maximal möglichen Schädigung bei einer gegebenen Pulsenergie- oder SAR-Folge.

Gemäß der vorliegenden Erfindung wird diese Aufgabe durch Verfahren zur Steuerung eines Hochfrequenzverstärkers und entsprechend ausgestaltete Magnetresonanzanlagen gemäß der unabhängigen Ansprüche gelöst. Die abhängigen Ansprüche definieren Ausführungsformen der Erfindung.

Die vorliegende Erfindung stellt ein Verfahren zur Steuerung eines Hochfrequenzverstärkers einer Magnetresonanzanlage bereit. Bei dem Verfahren wird eine von dem Hochfrequenzverstärker abzugebende Hochfrequenzleistung während einer Untersuchung eines Patienten in der Magnetresonanzanlage bestimmt. Unter Verwendung einer Lambertschen Funktion wird eine Maximaltemperatur im Gewebe des Patienten in Abhängigkeit von der Hochfrequenzleistung bestimmt. Der Hochfrequenzverstärker wird in Abhängigkeit von der Maximaltemperatur eingestellt. Beispielsweise kann der Hochfrequenzverstärker bei Erreichen der Maximaltemperatur im Gewebe abgeschaltet werden oder eine Leistung des Hochfrequenzverstärkers für beispielsweise eine Pulsenergie- oder SAR-Folge kann so eingestellt werden, dass die Maximaltemperatur nicht überschritten wird.

Durch die Bestimmung der Maximaltemperatur unter Verwendung der Lambertschen Funktion kann eine exaktere Berechnung der maximalen Temperaturerhöhung und somit von CEM43-Werten erreicht werden. Dadurch kann die Leistungsfähigkeit der Magnetresonanzanlage verbessert werden, wodurch kürzere Messzeiten, bessere Bildqualität oder mehr Bildschichten und damit eine bessere Diagnose erreicht werden können. Ferner können Gewebeschädigungen durch zu hohe Temperaturen zuverlässig vermieden werden.

Die Maximaltemperatur kann eine lokale Maximaltemperatur in einem Gewebeabschnitt des Patienten sein. Der Gewebeabschnitt kann beispielsweise wenige Kubikzentimeter umfassen, beispielsweise 1 cm$^3$ bis 10 cm$^3$. In diesem Fall wird eine von dem Hochfrequenzverstärker abzugebende lokale Hochfrequenzleistung in dem Gewebeabschnitt während der Untersuchung des Patienten in der Magnetresonanzanlage bestimmt. Abhängig von dieser lokalen Hochfrequenzleistung wird unter Verwendung der Lambertschen Funktion die Maximaltemperatur in dem Gewebeabschnitt des Patienten bestimmt.

Die Bestimmung der Maximaltemperatur in dem Gewebeabschnitt unter Verwendung der Lambertschen Funktion bietet im Gegensatz zu Näherungsformeln eine höhere Genauigkeit, sodass Sicherheitszuschläge, welche bei Näherungsformeln notwendig sind, nicht erforderlich sind oder zumindest deutlich geringer sein können. Im Vergleich zu Simulationsrechnungen, welche eine verhältnismäßig lange Rechenzeit benötigen, ist die Bestimmung der Maximaltemperatur in dem Gewebeabschnitt unter Verwendung der Lambertschen Funktion in Echtzeit in einer Magnetresonanzanlage realisierbar.

Insbesondere die Bestimmung der lokalen Maximaltemperatur kann in Abhängigkeit von einem lokalen Parameter für eine Gefäßerweiterung, einer Masse des Gewebeabschnitts und/oder einem thermischen Leitwert aufgrund einer Perfusion in dem Gewebeabschnitt durchgeführt werden. Insbesondere das Gefäßsystem kann bei der Regulation des thermischen Haushaltes eine signifikante Rolle spielen. Eine Wärmeleitungsmodellierung in blutdurchströmtem Gewebe ist beispielsweise aus Pennes Bioheat-Gleichung bekannt (H. H. Pennes; Analysis of tissue and arterial blood temperatures in the resting human forearm; Journal of Applied Physiology, 1:93-122, 1948). In einer allgemeinen Form lautet Pennes Bioheat-Gleichung:

$$c\varrho\,\frac{\partial T}{\partial t} = \kappa\nabla^2 T - c_b\omega(T - T_b) + \varrho S + M \qquad (2)$$

Die Berechnung der Temperatur aus dieser Gleichung erfordert eine Simulation oder ein Modell. Unter Verwendung einer Lambertschen Funktion kann hingegen die lokale Maximaltemperatur unter Berücksichtigung des thermischen Leitwerts aufgrund der Perfusion in dem lokalen Gewebeabschnitt direkt berechnet werden. Somit ist eine einfache und exakte Bestimmung der lokalen Maximaltemperatur möglich.

Die lokale Maximaltemperatur $T_{max}$ kann beispielsweise unter Verwendung der Lambertschen Funktion $W_0$ gemäß der Gleichung:

$$T_{max} = \frac{\Delta B}{\log(2)}\cdot W_0\left(\frac{S\,m\,\log(2)}{G_{Perf0}\,\Delta B}\right) \qquad (3)$$

bestimmt werden. Dabei bezeichnet $S$ die lokale Hochfrequenzleistung in dem Gewebeabschnitt, $\Delta B$ den lokalen Parameter für die Gefäßerweiterung in dem Gewebeabschnitt, $m$ die Masse des Gewebeabschnitts, und $G_{Perf0}$ den thermischen Leitwert aufgrund der Perfusion in dem Gewebeabschnitt.

Die vorliegende Erfindung stellt ein weiteres Verfahren zur Steuerung eines Hochfrequenzverstärkers einer Magnetresonanzanlage bereit. Bei dem Verfahren wird eine von dem Hochfrequenzverstärker während einer Untersuchung eines Patienten in der Magnetresonanzanlage abzugebende Hochfrequenzleistung bestimmt. Ferner wird bei dem Verfahren ein zeitlicher Temperaturverlauf im Gewebe des Patienten bestimmt. Der Temperaturverlauf umfasst zumindest eine vorhergehende Temperatur in dem Gewebe zu einem vorhergehenden Zeitpunkt und eine aktuelle Temperatur in dem Gewebe zu einem aktuellen Zeitpunkt. Die aktuelle Temperatur wird in Abhängigkeit von der vorhergehenden Temperatur und in Abhängigkeit von der Hochfrequenzleistung zu dem aktuellen Zeitpunkt bestimmt. Der Hochfrequenzverstärker wird in Abhängigkeit von dem Temperaturverlauf eingestellt. Mit dem Verfahren können daher Temperaturerhöhungen auf einfache Art und Weise bestimmt werden. Auf der Grundlage des Temperaturverlaufs kann beispielsweise ein CEM43-Wert bestimmt werden, um beispielsweise bei einer Überschreitung eines Grenzwertes für den CEM43-Wert den Hochfrequenzverstärker während der Magnetresonanzuntersuchung abzuschalten oder bereits vor Beginn der Magnetresonanzuntersuchung eine entsprechende Warnung auszugeben.

Der Temperaturverlauf kann einen lokalen Temperaturverlauf in einem Gewebeabschnitt des Patienten umfassen. Zum Bestimmen der von dem Hochfrequenzverstärker abzugebenden Hochfrequenzleistung wird eine von dem Hochfrequenzverstärker abzugebende lokale Hochfrequenzleistung in dem Gewebeabschnitt während der Untersuchung des Patienten in der Magnetresonanzanlage bestimmt. Der lokale Temperaturverlauf, welcher beispielsweise eine vorhergehende Temperatur in dem Gewebeabschnitt zu dem vorhergehenden Zeitpunkt und eine aktuelle Temperatur in dem Gewebeabschnitt zu dem aktuellen Zeitpunkt umfasst, kann, wie zuvor beschrieben, iterativ unter Berücksichtigung der lokalen Hochfrequenzleistung bestimmt werden.

Bei der Bestimmung des lokalen Temperaturverlaufs können ferner eine oder mehrere der folgenden Parameter berücksichtigt werden: ein lokaler Parameter für die Gefäßerweiterung in dem Gewebeabschnitt, eine Masse des Gewebeabschnitts, ein thermischer Leitwert aufgrund der Perfusion in dem Gewebeabschnitt, eine Zeitdifferenz zwischen dem aktuellen Zeitpunkt und dem vorhergehenden Zeitpunkt sowie eine Wärmekapazität des Gewebeabschnitts.

Wie zuvor beschrieben, kann insbesondere das Gefäßsystem bei der Regulation des thermischen Haushaltes eine signifikante Rolle spielen. Die Berechnung der Temperatur und somit des Temperaturverlauf aus der zuvor beschriebenen Pennes Bioheat Gleichung erfordert eine Simulation oder ein Modell. Durch die iterative Bestimmung des zeitlichen Temperaturverlaufs kann hingegen der lokale Temperaturverlauf unter Berücksichtigung des thermischen Leitwerts aufgrund der Perfusion in dem lokalen Gewebeabschnitt berechnet werden. Somit ist eine einfache und verhältnismäßig genaue Bestimmung des lokalen Temperaturverlaufs möglich.

Die aktuelle Temperatur $T_n$ zum dem aktuellen Zeitpunkt kann gemäß der Gleichung:

$$T_n = T_{n-1} + \frac{\Delta t \cdot G_{Perf0}}{C} \cdot \left( \frac{S\,m}{G_{Perf0}\,\Delta B} - 2^{\frac{T_{n-1}}{\Delta B}} T_{n-1} \right) \qquad (4)$$

bestimmt werden. Dabei bezeichnet $S$ die lokale Hochfrequenzleistung in dem Gewebeabschnitt, $\Delta B$ den lokalen Parameter für die Gefäßerweiterung in dem Gewebeabschnitt, $m$ die Masse des Gewebeabschnitts, $G_{Perf0}$ den thermischen Leitwert aufgrund der Perfusion in dem Gewebeabschnitt, $T_{n-1}$ die vorhergehende Temperatur zu dem vorhergehenden Zeitpunkt, $\Delta t$ die Zeitdifferenz zwischen dem aktuellen Zeitpunkt und dem vorhergehenden Zeitpunkt und $C$ die Wärmekapazität des Gewebeabschnitts.

Die vorliegende Erfindung betrifft weiterhin eine Magnetresonanzanlage, welche ein Gradientenfeldsystem, eine Hochfrequenzantenne, einen Hochfrequenzverstärker und eine Steuereinrichtung umfasst. Die Magnetresonanzanlage kann weitere Komponenten umfassen, beispielsweise einen Grundfeldmagneten sowie eine Bedienvorrichtung für die Magnetresonanzanlage. Die Steuereinrichtung dient zur Steuerung des Gradientenfeldsystems und des Hochfrequenzverstärkers, zum Empfang der von der Hochfrequenzantenne aufgenommenen Messsignale, zur Auswertung der Messsignale und zur Erstellung von Magnetresonanzdaten. Die Magnetresonanzanlage ist ausgestaltet, eine von einem Hochfrequenzverstärker der Magnetresonanzanlage während einer Untersuchung eines Patienten in der Magnetresonanzanlage abzugebende Hochfrequenzleistung zu bestimmen und eine Maximaltemperatur im Gewebe des Patienten in Abhängigkeit von der Hochfrequenzleistung unter Verwendung einer Lambertschen Funktion zu bestimmen. In Abhängigkeit von der Maximaltemperatur wird der Hochfrequenzverstärker eingestellt und gesteuert. Das Bestimmen der von dem Hochfrequenzverstärker abzugebenden Hochfrequenzleistung, das Bestimmen der Maximaltemperatur im Gewebe des Patienten und das Einstellen des Hochfrequenzverstärkers können beispielsweise von der Steuereinrichtung durchgeführt werden. Die Steuereinrichtung kann dazu eine elektronische Steuereinrichtung, beispielsweise einen Mikroprozessor, sowie entsprechende Steuerinformationen, beispielsweise ein Steuerprogramm, umfassen. Die Magnetresonanzanlage ist daher zur Durchführung des zuvor beschriebenen Verfahrens und seiner Ausführungsformen geeignet und umfasst somit auch die im Zusammenhang mit dem Verfahren zuvor beschriebenen Vorteile.

Die vorliegende Erfindung betrifft eine weitere Magnetresonanzanlage, welche ein Gradientenfeldsystem, eine Hochfrequenzantenne, einen Hochfrequenzverstärker und eine Steuereinrichtung umfasst. Die Steuereinrichtung dient zur Steuerung des Gradientenfeldsystems und des Hochfrequenzverstärkers, zum Empfang der von der Hochfrequenzantenne aufgenommenen Messsignale, zur Auswertung der Messsignale und zur Erstellung von Magnetresonanzdaten. Die Magnetresonanzanlage ist ausgestaltet, eine von einem Hochfrequenzverstärker der Magnetresonanzanlage während einer Untersuchung eines Patienten in der Magnetresonanzanlage abzugebende Hochfrequenzleistung zu bestimmen und einen zeitlichen Temperaturverlauf Gewebe des Patienten zu bestimmen. Der zeitliche Temperaturverlauf umfasst zumindest eine vorhergehende Temperatur in dem Gewebe zu einem vorhergehenden Zeitpunkt und eine aktuelle Temperatur in dem Gewebe zu einem aktuellen Zeitpunkt. Der Temperaturverlauf kann weitere Temperaturen zu vorhergehenden Zeitpunkten umfassen. Die aktuelle Temperatur wird in Abhängigkeit von der Hochfrequenzleistung zu dem aktuellen Zeitpunkt und in Abhängigkeit von der vorhergehenden Temperatur bestimmt. In Abhängigkeit von dem Temperaturverlauf wird der Hochfrequenzverstärker gesteuert und eingestellt. Das Bestimmen der von dem Hochfrequenzverstärker abzugebenden Hochfrequenzleistung, das iterative Bestimmen des Temperaturverlaufs und das Einstellen des Hochfrequenzverstärkers können beispielsweise von der Steuereinrichtung durchgeführt werden. Die Steuereinrichtung kann dazu eine elektronische Steuereinrichtung, beispielsweise einen Mikroprozessor, sowie entsprechende Steuerinformationen, beispielsweise ein Steuerprogramm, umfassen. Die Magnetresonanzanlage ist daher zur Durchführung des zuvor beschriebenen Verfahrens und seiner Ausführungsformen geeignet und umfasst somit auch die im Zusammenhang mit dem Verfahren zuvor beschriebenen Vorteile.

Weiterhin wird gemäß der vorliegenden Erfindung ein Computerprogramm bereitgestellt, welches in einen Speicher einer programmierbaren Steuereinrichtung einer Magnetresonanzanzanlage geladen werden kann. Mit diesem Computerprogramm können alle oder verschiedene der zuvor beschriebenen Ausführungsformen des erfindungsgemäßen Verfahrens ausgeführt werden, wenn das Computerprogramm in der programmierbaren Steuereinrichtung läuft. Dabei benötigt das Computerprogramm eventuell Programmmittel, zum Beispiel Bibliotheken oder Hilfsfunktionen, um die entsprechenden Ausführungsformen des Verfahrens zu realisieren. Anders ausgedrückt soll mit dem auf das Computerprogramm gerichteten Anspruch insbesondere ein Computerprogramm oder eine Software unter Schutz gestellt werden, mit welcher eine der oben beschriebenen Ausführungsformen des erfindungsgemäßen Verfahrens ausgeführt werden kann, beziehungsweise welche diese Ausführungsform ausführt. Dabei kann es sich bei der Software um einen Quellcode, zum Beispiel C++, welcher noch kompiliert und übersetzt und gebunden, oder welcher nur interpretiert werden muss, oder um einen ausführbaren Softwarecode handeln, welcher zur Ausführung nur noch in die entsprechende Steuereinrichtung zu laden ist.

Schließlich stellt die vorliegende Erfindung einen elektronisch lesbaren Datenträger, beispielsweise eine DVD, ein Magnetband oder einen USB-Stick bereit, auf welchem elektronisch lesbare Steuerinformationen, insbesondere Software, wie sie zuvor beschrieben wurde, gespeichert sind. Wenn diese Steuerinformationen von dem Datenträger gelesen und in einer Steuereinrichtung gespeichert werden, können alle erfindungsgemäßen Ausführungsformen des beschriebenen Verfahrens ausgeführt werden.

Die vorliegende Erfindung wird nachfolgend unter Bezugnahme auf die Zeichnungen anhand bevorzugter Ausführungsformen erläutert werden.

Fig. 1 zeigt schematisch eine Magnetresonanzanlage gemäß einer Ausführungsform der vorliegenden Erfindung.

Fig. 2 zeigt schematisch ein elektrisches Ersatzschaltbild für eine Wärmestromgleichung in einem stabilen Ausgangszustand ohne eingestrahlte Hochfrequenzleistung.

Fig. 3 zeigt schematisch ein elektrisches Ersatzschaltbild für eine Wärmestromgleichung mit eingestrahlter Hochfrequenzleistung.

Fig. 4 zeigt schematisch ein elektrisches Ersatzschaltbild für eine Wärmestromgleichung für Ganzkörpererwärmung und lokale Erwärmung.

Fig. 5 zeigt schematisch ein elektrisches Ersatzschaltbild für eine Wärmestromgleichung für Ganzkörpererwärmung und lokale Erwärmung unter Berücksichtigung einer Thermoregulation.

Fig. 6 zeigt schematisch eine Perfusionsänderung für normale Thermoregulation und eingeschränkte Thermoregulation.

Fig. 7 zeigt schematisch ein elektrisches Ersatzschaltbild für eine Wärmestromgleichung für lokale Erwärmung unter Berücksichtigung einer Thermoregulation.

Fig. 8 zeigt schematisch eine lokale SAR Belastung über der Zeit während beispielsweise einer Magnetresonanzuntersuchung.

Fig. 9 zeigt schematisch einen Vergleich eines Temperaturanstiegs bei normaler Thermoregulation gemäß einem Runge-Kutta-Näherungsverfahren, unter Verwendung einer Wurzelannäherung und unter Verwendung einer iterativen Approximation gemäß einer Ausführungsform der vorliegenden Erfindung.

Fig. 10 zeigt schematisch einen Vergleich eines Temperaturanstiegs bei eingeschränkter Thermoregulation gemäß einem Runge-Kutta Näherungsverfahren, unter Verwendung einer Wurzelannäherung und unter Verwendung einer iterativen Approximation gemäß einer Ausführungsform der vorliegenden Erfindung.

Fig. 11 zeigt schematisch einen Vergleich einer CEM43 bei normaler Thermoregulation gemäß einem Runge-Kutta-Näherungsverfahren, unter Verwendung einer Wurzelannäherung und unter Verwendung einer iterativen Approximation gemäß einer Ausführungsform der vorliegenden Erfindung.

Fig. 12 zeigt schematisch einen Vergleich einer CEM43 bei eingeschränkter Thermoregulation gemäß einem Runge-Kutta-Näherungsverfahren, unter Verwendung einer Wurzelannäherung und unter Verwendung einer iterativen Approximation gemäß einer Ausführungsform der vorliegenden Erfindung.

Figur 13 zeigt schematisch ein Verfahren für eine Magnetresonanzanlage gemäß einer Ausführungsform der vorliegenden Erfindung, um eine Schädigung eines Patienten bei einer Untersuchung in der Magnetresonanzanlage zu vermeiden.

Figur 14 zeigt schematisch ein weiteres Verfahren für eine Magnetresonanzanlage gemäß einer Ausführungsform der vorliegenden Erfindung, um eine Schädigung eines Patienten bei einer Untersuchung in der Magnetresonanzanlage zu vermeiden.

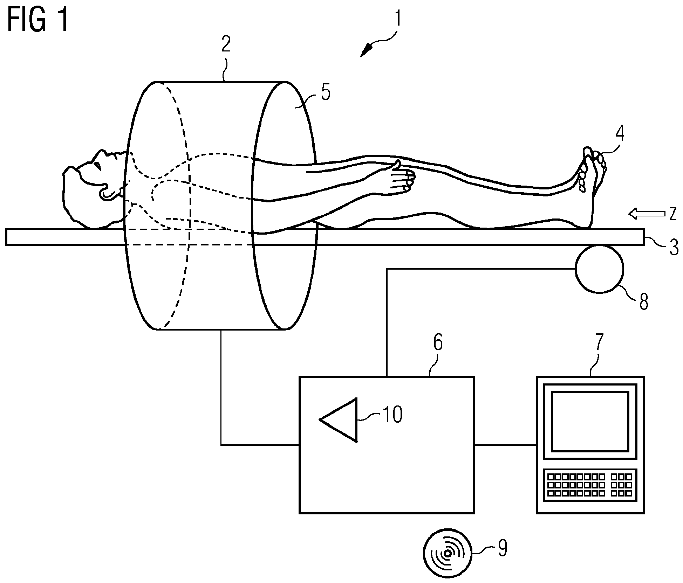

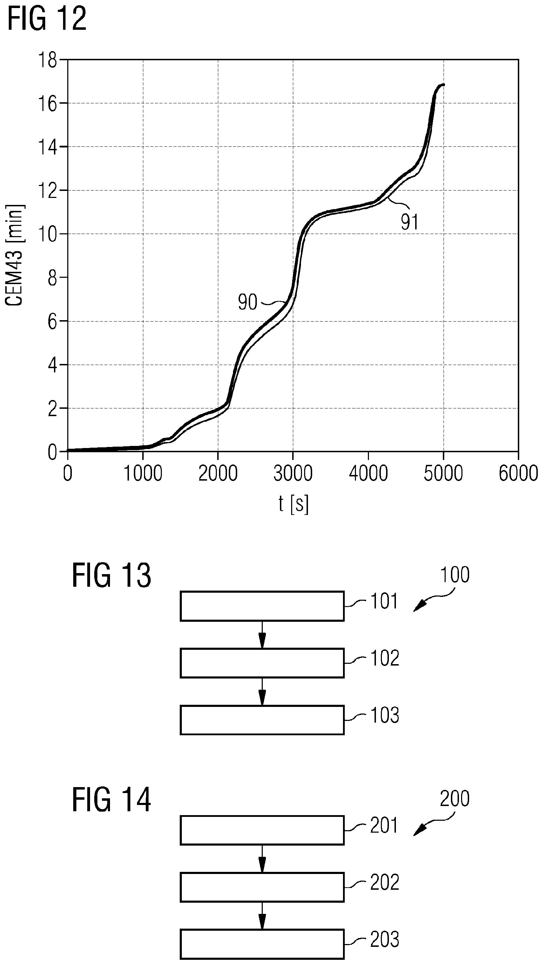

Figur 1 zeigt eine schematische Darstellung einer Magnetresonanzanlage 1. Die Magnetresonanzanlage 1 umfasst einen Tomographen 2, einen Untersuchungstisch 3 für eine Untersuchungsperson 4, welche auf dem Untersuchungstisch 3 durch eine Öffnung 5 des Tomographen 2 bewegt werden kann, eine Steuereinrichtung 6, eine Auswertevorrichtung 7 und eine Antriebseinheit 8. Die Steuereinrichtung 6 steuert den Tomographen 2 an und empfängt Signale von dem Tomographen 2, welche von den Tomographen 2 aufgenommen werden. Zur Erzeugung der Magnetresonanzdaten weist der Tomograph 2 einen nicht gezeigten Grundfeldmagneten auf, welcher ein Grundmagnetfeld B0 erzeugt, sowie ein nicht gezeigtes Gradientenfeldsystem zur Erzeugung von Gradientenfeldern. Weiterhin umfasst der Tomograph 2 eine oder mehrere Hochfrequenzantennen zur Erzeugung von Hochfrequenzsignalen und zum Empfangen von Messsignalen, welche von der Steuereinrichtung 6 und der Auswertevorrichtung 7 zur Erzeugung von Magnetresonanzbildern verwendet werden. Die Hochfrequenzantennen werden dabei zur Erzeugung der Hochfrequenzsignale von einem Hochfrequenzverstärker 10 angesteuert. Der Hochfrequenzverstärker 10 wird wiederum von der Steuereinrichtung 6 angesteuert. Die Steuereinrichtung 6 steuert weiterhin die Antriebseinheit 8 an, um den Untersuchungstisch 3 entlang einer Richtung Z zusammen mit der Untersuchungsperson 4 durch die Öffnung 5 des Tomographen 2 zu bewegen. Die Steuereinrichtung 6 und die Auswertevorrichtung 7 können beispielsweise ein Computersystem mit einem Bildschirm, einer Tastatur und einem Datenträger 9 umfassen, auf welchem elektronisch lesbare Steuerinformationen gespeichert sind, welche derart ausgestaltet sind, dass sie bei einer Verwendung des Datenträgers 9 in der Auswertevorrichtung 7 und der Steuereinrichtung 6 die unter Bezugnahme auf Figuren 13 und 14 beschriebenen Verfahren durchführen.

Um zu gewährleisten, dass eine Belastung der Untersuchungsperson (Patient) mit elektromagnetischen Feldern unter zulässigen Grenzen bleibt, können für verschiedene Körperregionen und Betriebsarten mit einem Computerprogramm entsprechende Grenzwerte bestimmt werden und einer Online-Überwachungseinheit, welche als Teil der Steuereinrichtung 6 ausgebildet sein kann, übergeben werden. Die Online-Überwachungseinheit bestimmt beispielsweise die abgegebene Hochfrequenzleistung mittels Spannungsmessung an dem Hochfrequenzverstärker 10. Davon kann die in den Sendevorrichtungen (Hochfrequenzantennen) verbleibende Verlustleistung abgezogen werden. Unter Verwendung der Masse des Patienten 4 kann daraus eine aktuelle spezifische Absorptionsrate (SAR) bestimmt werden und mit den entsprechenden Grenzwerten verglichen werden. Wird der zulässige Grenzwert für die spezifische Absorptionsrate überschritten, so wird der Hochfrequenzverstärker 10 abgeschaltet und somit die Durchführung der Magnetresonanzuntersuchung abgebrochen. Bei Sendespulen, welche kein homogenes B1-Feld erzeugen, kann die lokale SAR begrenzt werden.

Simulationsrechnungen haben jedoch gezeigt, dass auch bei einem homogenen B1 Feld die lokale SAR über dem in der Norm festgelegten Grenzwert liegen kann.

Daher gibt es Bestrebungen, die Gewebeschädigung durch Wärme exakter zu bestimmen. Ein möglicher Ansatz dafür ist die Berechnung des CEM43-Wertes (CEM43: Cumulative Equivalent Minutes at 43 Degree Celsius). Der CEM43-Wert kann aus dem Schädigungsintegral von Arrhenius abgeleitet werden zu:

$$CEM43(\tau) = \int_0^\tau R^{T_{start}+T(t)-43°C} dt \qquad (5)$$

Die globale SAR als auch die lokale SAR können bekannt sein. Zur Bestimmung des CEM43-Werts ist jedoch die konkrete Temperatur $T(t)$ im Gewebe erforderlich.

In einer allgemeinen Form kann diese mittels Pennes Bioheat-Gleichung bestimmt werden:

$$cQ\frac{\partial T}{\partial t} = \kappa\nabla^2 T - c_b\omega(T - T_b) + \varrho S + M \qquad (6)$$

mit:

$c$ - spezifische Wärmekapizität [Ws/(kgK)]
$\kappa$ - thermische Leitfähigkeit [W/(mK)]
$\omega$ - Perfusionsrate [kg/(m$^3$s)]
$\varrho$ - Dichte [kg/m$^3$]
$T$ - Temperatur [K]
$S$ - SAR [W/kg]
$M$ - metabolische Rate pro Volumeneinheit = $\Phi/V$ [W/m$^3$]
$\Phi$ - Wärmestrom [W]
$V$ - Volumen [m$^3$]

Der Index $b$ bezeichnet dabei Parameter, welche sich auf das Blut beziehen.

Die Berechnung der Temperatur aus Pennes Bioheat-Gleichung (6) erfordert eine Simulation oder ein Modell.

Diese Gleichung (6) kann in eine Wärmestromgleichung umgeformt werden und ermöglicht somit eine Betrachtung der Leistungsbilanz:

$$cm\frac{\partial T}{\partial t} = \frac{m}{\varrho}\kappa\nabla^2 T - c_b\omega\frac{m}{\varrho}(T - T_b) + mS + \Phi \qquad (7)$$

Die Wärmestromgleichung (7) lässt sich mit der folgenden Analogiebetrachtung in eine analoge elektrische Ersatzschaltung umwandeln:

Die Temperatur $T$ entspricht der elektrischen Spannung $U$. Der Wärmestrom $\Phi$ entspricht dem elektrischen Strom $I$. Der thermische Widerstand $R$ entspricht dem elektrischen Widerstand $R$.
Der thermische Leitwert $G$ entspricht dem elektrischen Leitwert $G$. Die Wärmeleitfähigkeit $\kappa$ entspricht der elektrischen Leitfähig $\kappa$. Die Wärmekapazität $C_{th}=cm$ entspricht der elektrischen Kapazität $C$.

Der thermische Leitwert $G_{cond}$ des Gewebes ergibt sich aus der Wärmestromgleichung (7) demnach zu:

$$G_{cond} = -\frac{m\kappa\nabla^2 T}{\varrho T} \qquad (8)$$

Der thermische Leitwert $G_{perf}$ durch die Perfusion des Bluts durch das Gewebe ergibt sich aus der Wärmestromgleichung (7) zu:

$$G_{perf} = \frac{c_b\omega m}{\varrho} \qquad (9)$$

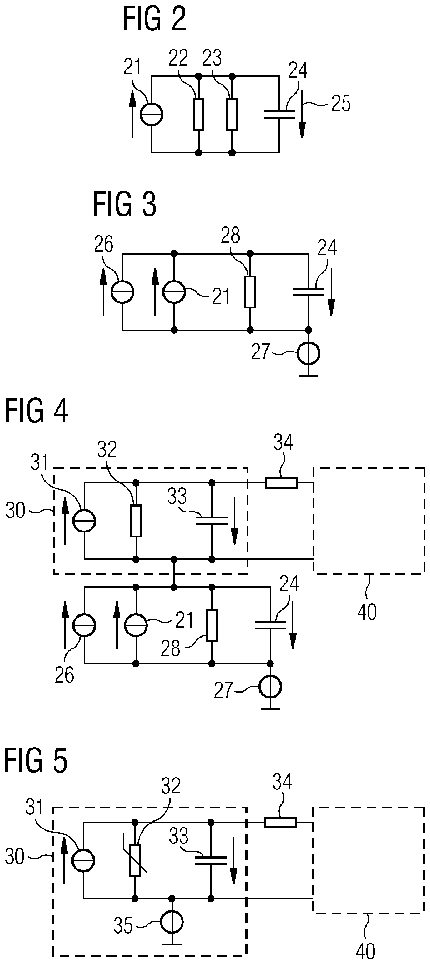

Fig. 2 zeigt ein entsprechendes elektrisches Ersatzschaltbild mit einer elektrischen Stromquelle 21, einem ersten Leitwert 22, einem zweiten Leitwert 23 und einer Kapazität 24. Die elektrische Stromquelle 21 erzeugt einen elektrischen Strom $I$, welcher dem thermischen Wärmestrom $\Phi$ entspricht. Der erste Leitwert 22 stellt den thermischen Leitwert $G_{perf}$ durch die Perfusion des Bluts durch das Gewebe dar und der zweite Leitwert 23 stellt den thermischen Leitwert $G_{cond}$ des Gewebes das. Die Kapazität 24 repräsentiert die Wärmekapazität $C_{th}$ bzw. die entsprechende elektrische Kapazität $C$. Eine Spannung $U$ (dargestellt durch den Spannungspfeil 25) über der Kapazität 24 stellt somit die Gewebetemperatur $T$ dar.

Für diese in Fig. 2 gezeigte elektrische Ersatzschaltung gilt:

$$\frac{dU}{dt} = -\frac{G_{perf}+G_{cond}}{C}U + \frac{I}{C} \qquad (10)$$

Für den linearen Fall lautet die Lösung dieser Gleichung:

$$U(t) = \frac{I(1-e^{-\frac{t}{\tau}})}{G_{perf}+G_{cond}} + U(t=0)e^{-\frac{t}{\tau}} \qquad (11)$$

mit

$$\tau = \frac{C}{G_{perf}+G_{cond}} \qquad (12)$$

Ausgehend von einem thermisch stabilen Zustand ohne eingestrahlte Hochfrequenzleistung, d.h. bei SAR=0, hat das Gewebe die Temperatur des Blutes. Die Perfusion des Blutes hat somit keinen Einfluss auf die Temperatur und der metabolische Grundumsatz $\Phi$ ist so hoch, dass sich die Körperstammtemperatur von ca. 36,6 °C einstellt. Es wird davon ausgegangen, dass der metabolische Grundumsatz konstant bleibt.

Dieser Ausgangszustand wird durch eine Magnetresonanzuntersuchung gestört. Durch die eingestrahlte Hochfrequenzleistung wird ein zusätzlicher Wärmestrom bewirkt. Die eingestrahlte Ganzkörper SAR führt zu einer Erhöhung der Körperstammtemperatur. Im Allgemeinen führt eine Ganzkörper SAR von 4 W/kg zu einer maximalen Temperaturerhöhung von 1°. Das bedeutet, dass der mittlere Faktor $m/G_{cond}$ ca. 0,25 K/(W/kg) beträgt.

Fig. 3 zeigt ein entsprechendes elektrisches Ersatzschaltbild für den Fall einer bei einer Magnetresonanzuntersuchung eingestrahlten Ganzkörperhochfrequenzleistung von $P=SAR*m$. Im Ersatzschaltbild wird die Ganzkörperhochfrequenzleistung P als zusätzliche Stromquelle 26 dargestellt. Zusätzlich ist eine Spannungsquelle 27 in dem elektrischen Ersatzschaltbild der Fig. 3 dargestellt, welche eine Temperaturabweichung zur Umgebungstemperatur darstellt. Wenn die Umgebungstemperatur stark abweicht, kann es zu einer entsprechenden Ausgleichsreaktion des Körpers, wie zum Beispiel schwitzen oder frösteln, kommen, wodurch der Leitwert $G_{cond}$ nichtlinearer wird. Der Leitwert 28 umfasst zur Vereinfachung sowohl den thermischen Leitwert des Gewebes als auch den thermischen Leitwert durch die Perfusion des Blutes.

Darüber hinaus kann eine lokal eingestrahlte Hochfrequenzleistung bei einer Magnetresonanzuntersuchung vorhanden sein. Fig. 4 zeigt ein entsprechendes elektrisches Ersatzschaltbild für eine lokale Temperaturerhöhung in einem Bereich 30 mit der zuvor in Verbindung mit Fig. 3 gezeigten Ganzkörperhochfrequenzleistung (Bezugszeichen 21, 24, 26, 27, 28).

In dem lokalen Bereich 30 bewirkt die lokale SAR eine Hochfrequenzleistungseinspeisung, welche durch die Stromquelle 31 dargestellt wird. Die lokale Temperatur stellt sich an der Kapazität 33 ein. In dem lokalen Bereich 30 wirkt der Leitwert 32 durch die Perfusion des Blutes. Der Leitwert 34 stellt den thermischen Leitwert zu Nachbargewebe 40 in der Umgebung des lokalen Bereichs 30 dar. In dem lokalen Bereich 30 stellt sich über der Kapazität 33 eine Temperaturerhöhung $T_{inc}$ aufgrund der lokalen SAR ein. Durch den Wärmefluss in das Nachbargewebe 40 kann in dem Nachbargewebe 40 ebenfalls eine Temperaturerhöhung $T_{inc2}$ auftreten.

Für den Fall, dass sich der lokale Bereich 30, auf welchen die lokale SAR wirkt, in einem Umgebungsbereich mit gleicher Temperatur befindet, d.h. $T_{inc}=T_{inc2}$, findet über die thermische Leitfähigkeit des Gewebes (Leitwert 34) kein Temperaturaustausch statt. In diesem Fall kann die Thermoregulation die Perfusion des Blutes beeinflussen, sodass der Leitwert 32 von der Temperatur $T_{inc}$ abhängt und somit nichtlinearer wird. Fig. 5 zeigt ein entsprechendes Ersatzschaltbild für den Bereich 30 mit dem temperaturabhängigen Leitwert 32. Die Spannungsquelle 35 repräsentiert in diesem Fall die Bluttemperatur.

Bei der Thermoregulation über die Perfusion des Blutes ist zwischen Patienten mit normaler Thermoregulation und Patienten mit eingeschränkter Thermoregulation, wie zum Beispiel älteren Patienten oder an Diabetes erkrankten Patienten, zu unterscheiden.

Aufgrund der Thermoregulation ist die Perfusionsrate $\omega$ abhängig von der Temperatur $T$. Ausgehend von einer Perfusionsrate $\omega_0$ ohne Thermoregulation ergibt sich die temperaturabhängige Perfusionsrate $\omega(T)$ für die normale Thermoregulation zu:

$$\omega(T) = \omega_0 L_b(T) \qquad (13)$$

mit der Perfusionsänderung $L_b(T)$ für normale Thermoregulation. Die Perfusionsänderung $L_b(T)$ für normale Thermoregulation kann mittels eines lokalen Parameters $\Delta B$ für die Blutgefäßerweiterung beschrieben werden als:

$$L_b(T) = 1 \; f\ddot{u}r \; T < 37°C \qquad\qquad (14)$$

und

$$L_b(T) = 2^{\frac{T-37}{\Delta B}} \; f\ddot{u}r \; T \geq 37°C \qquad\qquad (15)$$

mit $\Delta B = 1{,}6K$ und einem Maximalwert für $L_b$ von 32 für Haut und einem Maximalwert für $L_b$ von 15 für sonstiges Gewebe.

Die temperaturabhängige Perfusionsrate $\omega(T)$ für die eingeschränkte Thermoregulation ist:

$$\omega(T) = \omega_0 L_{b\_imp}(T) \qquad\qquad (16)$$

mit der Perfusionsänderung $L_{b\_imp}(T)$ für eingeschränkte Thermoregulation. Die Perfusionsänderung $L_{b\_imp}(T)$ für eingeschränkte Thermoregulation kann auf der Grundlage der Perfusionsänderung $L_b(T)$ für normalen Thermoregulation beschrieben werden als:

$$L_{b\_imp}(T) = 1 + \big(0{,}3(L_b(T) - 1)\big) = 0{,}7 + 0{,}3 L_b(T) \qquad\qquad (17)$$

mit einem Maximalwert für $L_{b\_imp}$ von 10,3 für Haut und einem Maximalwert für $L_{b\_imp}$ von 5,2 für sonstiges Gewebe.

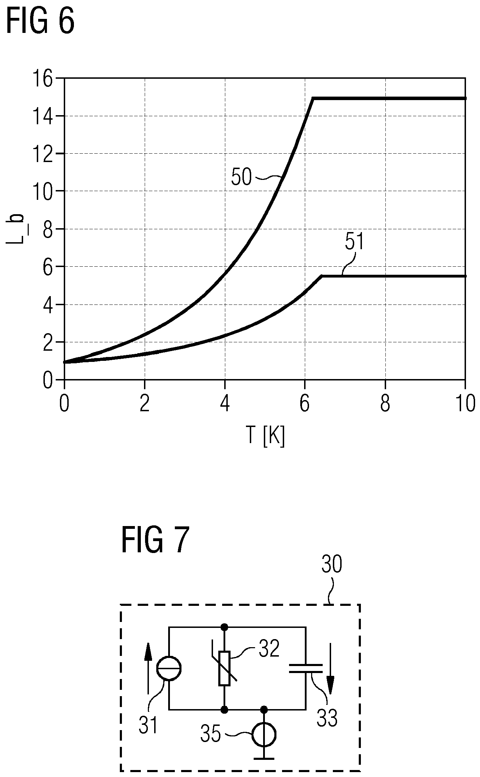

Fig. 6 zeigt beispielhaft den Verlauf der Perfusionsänderung für sonstiges Gewebe (also nicht für die Haut) für normale Thermoregulation mit dem Graph 50 und für die eingeschränkte Thermoregulation mit dem Graph 51.

Für das in Fig. 7 gezeigte elektrische Ersatzschaltbild für den lokalen Bereich 30 (vergleiche Fig. 5) mit dem temperaturabhängigen Leitwert 32 ergibt sich somit aus Gleichung (4) :

$$\frac{dU}{dt} = -\frac{G_{perf0} 2^{\frac{U}{\Delta B}}}{C} U + \frac{I}{C} \qquad\qquad (18)$$

Diese Differenzialgleichung ist nicht analytisch lösbar, kann aber mit Näherungsverfahren, zum Beispiel einem Runge-Kutta-Verfahren, näherungsweise berechnet werden oder mit einer Funktion der Form $U_{max}(1-e^{-t/\tau})$ angenähert werden.

Für eine Magnetresonanzuntersuchung und zum Patientenschutz ist im Wesentlichen interessant, wie hoch die maximale Erwärmung werden kann und wie die Erwärmung zeitlich verläuft.

Zur Bestimmung der maximalen Erwärmung kann beispielsweise eine Wurzelannäherung verwendet werden. Ein Vergleich dieser Wurzelannäherung mit dem wesentlich genaueren Näherungsverfahren nach Runge-Kutta zeigt jedoch, dass signifikante Ungenauigkeiten auftreten können, sodass bei Verwendung der Wurzelannäherung beträchtliche Sicherheitsaufschläge hinzuzurechnen sind.

Aus Fig. 7 ist jedoch ersichtlich, dass der Kondensator im zeitlich unendlichen auf die Maximalspannung $I/G_{perf}$ aufgeladen wird. Dies kann zur Bestimmung der Maximalspannung $U_{max}$, welche der Maximaltemperatur $T_{max}$ entspricht, wie folgt verwendet werden. Aus der Gleichung (18) zum Ersatzschaltbild der Fig. 7 ergibt sich:

$$U_{max} = \frac{I}{G_{perf0} 2^{\frac{U}{\Delta B}}} \qquad\qquad (19)$$

Diese Gleichung kann umgeformt werden zu:

$$U_{max} e^{\frac{U_{max}}{\Delta B} \log(2)} = \frac{I}{G_{perf0}} \qquad\qquad (20)$$

mit $x = \frac{U_{max}}{\Delta B} \log(2)$ folgt:

$$xe^x = \frac{I \log(2)}{G_{perf0} \, \Delta B} \qquad (21)$$

Eine Auflösung einer Gleichung $xe^x = y$ nach $x$ ist mit Hilfe der Lambertschen $W_0$-Funktion möglich: $X = W_0(y)$.

Durch Rücktransformation ergibt sich für die maximale elektrische Spannung $U_{max}$:

$$U_{max} = \frac{\Delta B}{\log(2)} \cdot W_0 \left( \frac{I \log(2)}{G_{Perf0} \, \Delta B} \right) \qquad (22)$$

bzw. für die maximale Temperatur $T_{max}$ entsprechend:

$$T_{max} = \frac{\Delta B}{\log(2)} \cdot W_0 \left( \frac{S \, m \log(2)}{G_{Perf0} \, \Delta B} \right) \qquad (23)$$

Durch die Verwendung der Lambertschen Funktion kann die lokale Maximaltemperatur unter Berücksichtigung des thermischen Leitwerts aufgrund der Perfusion in dem lokalen Gewebeabschnitt direkt berechnet werden. Somit ist eine einfache und exakte Bestimmung der lokalen Maximaltemperatur möglich.

Der zeitliche Verlauf der Temperatur kann beispielsweise numerisch als eine Superposition aus Erwärmung und Abkühlung berechnet werden. Dabei kann die oben genannte Wurzelannäherung als Basis verwendet werden:

$$T_{inc} = d\sqrt{cS} \qquad (24)$$

mit $d=1,3$ und $c=0,28K/(W/kg))$. Mit $\tau = \frac{640s}{1+\frac{\sqrt{cS}}{d}}$ folgt die Superposition aus Erwärmung und Abkühlung zu:

$$T_n = T_{inc} \left( 1 - e^{\frac{-\Delta t}{\tau}} \right) + T_{n-1} e^{\frac{-\Delta t}{\tau}} \qquad (25)$$

Durch annähern der e-Funktion als Taylorreihe 1. Grades folgt mit $e^{\frac{-\Delta t}{\tau}} \cong 1 - \frac{\Delta t}{\tau}$:

$$T_n = T_{inc} \frac{\Delta t}{\tau} + T_{n-1} \left( 1 - \frac{\Delta t}{\tau} \right) \qquad (26)$$

und Einsetzen der Wurzelannäherung:

$$T_n = T_{n-1} + \frac{\Delta t}{\tau} \left( d\sqrt{cS} - T_{n-1} \right) \qquad (27)$$

Ein Vergleich dieser Abschätzung des Temperaturverlaufs auf der Grundlage der Wurzelannäherung mit dem wesentlich genaueren Näherungsverfahren nach Runge-Kutter zeigt jedoch, dass signifikante Ungenauigkeiten auftreten können, sodass bei Verwendung dieser Abschätzung beträchtliche Sicherheitsaufschläge hinzuzurechnen sind.

Eine wesentlich genauere Approximation für die lokale Temperaturerhöhung kann auf der Grundlage der Differenzialgleichungen für einen lokalen Bereich, beispielsweise den Bereich 30, erreicht werden. Ausgehend von der obigen Differenzialgleichung (10) für das in Fig. 7 gezeigte Ersatzschaltbild folgt:

$$\frac{dU}{dt} = \frac{I}{C} - \frac{G_{perf0}2^{\frac{U}{\Delta B}}}{C}U \tag{28}$$

und weiter durch Vergrößern des Differenzial $d$ in ein Delta $\Delta$:

$$\Delta U = \Delta t(\frac{I}{C} - \frac{G_{perf0}2^{\frac{U}{\Delta B}}}{C}U) \tag{29}$$

Mit der Substitution $\tau_0 = \frac{C}{G_{perf0}}$ folgt:

$$\Delta U = U_n - U_{n-1} = \Delta t(\frac{I}{\tau_0 G_{perf0}} - \frac{2^{\frac{U_{n-1}}{\Delta B}}}{\tau_0}U_{n-1}) \tag{30}$$

und daraus folgt:

$$U_n = U_{n-1} + \frac{\Delta t}{\tau_0}(\frac{I}{G_{perf0}} - 2^{\frac{U_{n-1}}{\Delta B}}U_{n-1}) \tag{31}$$

Zurücktransformiert aus der elektrischen Ersatzschaltung in den Wärmestrom erhält man für die Temperatur $T_n$ zum Zeitpunkt $n$ mit der Nebenbedingung $\Delta t/\tau_0 \ll 1$ iterativ aus der Temperatur $T_{n-1}$ zum vorhergehenden Zeitpunkt $n-1$:

$$T_n = T_{n-1} + \frac{\Delta t}{\tau_0}(\frac{S\,m}{G_{perf0}} - 2^{\frac{U_{n-1}}{\Delta B}}T_{n-1}) \tag{32}$$

$\Delta t$ bezeichnet die Zeitdifferenz zwischen dem Zeitpunkt $n-1$ und dem Zeitpunkt $n$. Es ist zu beachten, dass die obige iterative Berechnungsvorschrift unabhängig von $\tau$ ist.

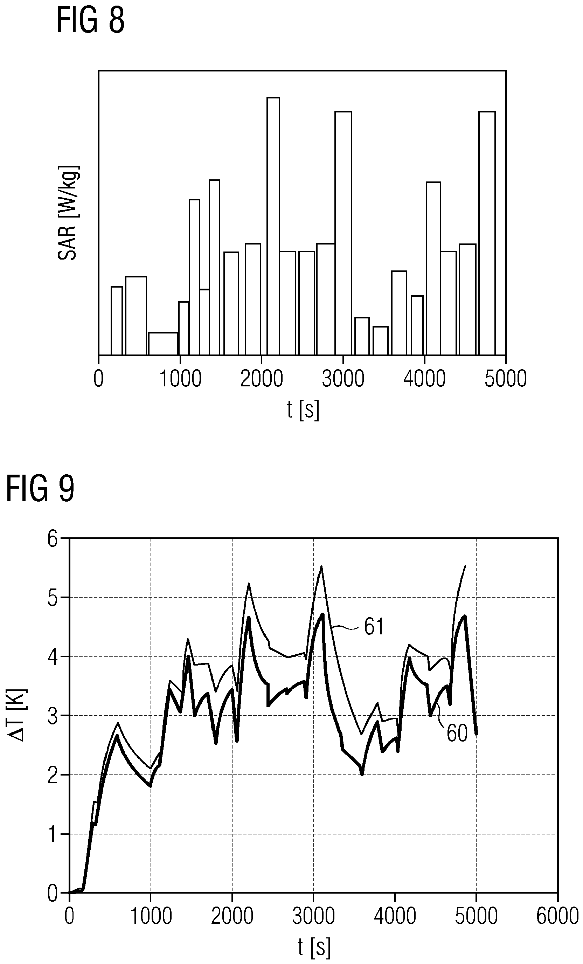

Fig. 8 zeigt schematisch eine lokale SAR Belastung über der Zeit, wie sie beispielsweise während einer Magnetresonanzuntersuchung durch eine eingestrahlte Hochfrequenzleistung in einem Gewebebereich eines Patienten auftreten kann.

Fig. 9 zeigt schematisch einen Vergleich eines Temperaturanstiegs infolge der in Fig. 8 gezeigten Einstrahlung bei normaler Thermoregulation gemäß einem Runge-Kutta-Näherungsverfahren für die Gleichung (18), unter Verwendung des zuvor beschriebenen Verfahrens mit Wurzelannäherung gemäß Gleichung (27) und unter Verwendung der zuvor beschriebenen iterativen Approximation gemäß Gleichung (32). Das Runge-Kutta-Näherungsverfahren und die Approximation gemäß Gleichung (32) resultieren im Wesentlichen in dem gleichen Graph 60 und sind daher in der Abbildung nicht unterscheidbar. Das Verfahren mit Wurzelannäherung gemäß Gleichung (27) liefert den Graph 61. Wie aus Fig. 9 ersichtlich ist, liefert das Runge-Kutta-Verfahren bzw. die Approximation gemäß Gleichung (32) einen Temperaturanstieg, welcher deutlich niedriger als der Temperaturanstieg gemäß dem Verfahren mit Wurzelannäherung gemäß Gleichung (27) ausfällt. Dies ermöglicht eine exaktere Berechnung der CEM 43, wodurch ein Leistungsvorteil bei Magnetresonanzuntersuchungen erreicht werden kann, der sich beispielsweise in einer kürzeren Messzeit, besserer Bildqualität oder mehr Bildschichten und damit besserer Diagnostizierbarkeit ausdrückt.

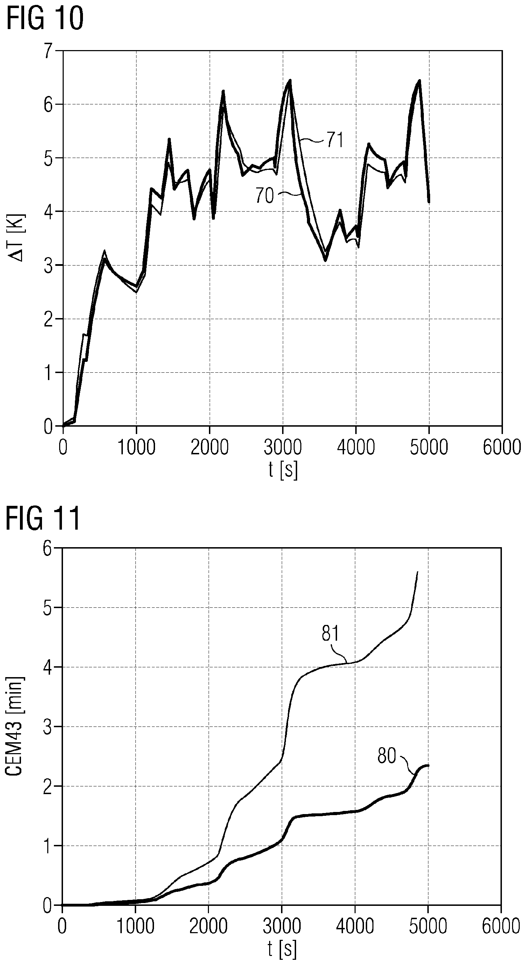

Fig. 10 zeigt schematisch einen Vergleich eines Temperaturanstiegs bei eingeschränkter Thermoregulation gemäß einem Runge-Kutta-Näherungsverfahren für die Gleichung (18), unter Verwendung des zuvor beschriebenen Verfahrens mit Wurzelannäherung gemäß Gleichung (27) und unter Verwendung der zuvor beschriebenen iterativen Approximation gemäß Gleichung (32) bei Einstrahlung gemäß Fig. 8. Das Runge-Kutta-Näherungsverfahren und die Approximation gemäß Gleichung (32) resultieren wiederum im Wesentlichen in dem gleichen Graph 70. Das Verfahren mit Wurzelannäherung gemäß Gleichung (27) lieferte den Graph 71. Wie aus Fig. 10 ersichtlich ist, liegt die Temperaturerhöhung gemäß Gleichung (27) (Wurzelannäherung) nicht immer über dem wesentlich genaueren Runge-Kutta-Näherungsverfahren gemäß Gleichung (18) bzw. der Approximation gemäß Gleichung (32), sodass insbesondere bei Patienten mit eingeschränkter Thermoregulation Sicherheitszuschläge bei der Verwendung der Temperaturerhöhung gemäß Gleichung (27) erforderlich sind, um eine Gewebeschädigung zu vermeiden.

Fig. 11 zeigt schematisch einen Vergleich einer CEM43 bei normaler Thermoregulation gemäß dem Runge-Kutta-Näherungsverfahren nach Gleichung (18), unter Verwendung der Wurzelannäherung nach Gleichung (27) und unter Verwendung der iterativen Approximation nach Gleichung (32). Das Runge-Kutta-Näherungsverfahren und die Approximation gemäß Gleichung (32) resultieren wiederum im Wesentlichen in dem gleichen Graph 80. Das Verfahren mit Wurzelannäherung gemäß Gleichung (27) liefert den Graph 81.

Fig. 12 zeigt schematisch einen Vergleich einer CEM43 bei eingeschränkter Thermoregulation gemäß dem Runge-Kutta-Näherungsverfahren nach Gleichung (18), unter Verwendung der Wurzelannäherung nach Gleichung (27) und unter Verwendung der iterativen Approximation nach Gleichung (32). Das Runge-Kutta-Näherungsverfahren und die Approximation gemäß Gleichung (32) resultieren wiederum im Wesentlichen in dem gleichen Graph 90. Das Verfahren mit Wurzelannäherung gemäß Gleichung (27) liefert den Graph 91.

Die zuvor beschriebenen Verfahren können beispielsweise im Vorfeld oder während einer Magnetresonanzuntersuchung von der Steuereinrichtung 6 der Magnetresonanzanlage 1 ausgeführt werden.

Wie in Fig. 13 gezeigt, kann die Steuereinrichtung 6 beispielsweise ein Verfahren 100 ausführen. In Schritt 101 wird eine von dem Hochfrequenzverstärker 10 abzugebende Hochfrequenzleistung während einer Untersuchung des Patienten 4 in der Magnetresonanzanlage 1 bestimmt. In Schritt 102 wird eine Maximaltemperatur $T_{max}$ im Gewebe des Patienten 4 in Abhängigkeit von der Hochfrequenzleistung unter Verwendung einer Lambertschen Funktion beispielsweise nach Gleichung (23) bestimmt. In Schritt 103 wird der Hochfrequenzverstärker 10 in Abhängigkeit von der Maximaltemperatur $T_{max}$ eingestellt, um beispielsweise eine Gewebeschädigung zu vermeiden.

Die Steuereinrichtung 6 kann ferner ein Verfahren 200, wie es in Fig. 14 gezeigt ist, ausführen. In Schritt 201 wird eine von dem Hochfrequenzverstärker 10 abzugebende Hochfrequenzleistung während einer Untersuchung des Patienten 4 in der Magnetresonanzanlage 1 bestimmt. In Schritt 202 wird ein zeitlicher Temperaturverlauf im Gewebe des Patienten 4 bestimmt, welcher zumindest eine vorhergehende Temperatur $T_{n-1}$ in dem Gewebe zu einem vorhergehenden Zeitpunkt $n-1$ und eine aktuelle Temperatur $T_n$ in dem Gewebe zu einem aktuellen Zeitpunkt $n$ umfasst. Die aktuelle Temperatur $T_n$ wird in Abhängigkeit von der vorhergehenden Temperatur $T_{n-1}$ und in Abhängigkeit von der Hochfrequenzleistung $S$ zu dem aktuellen Zeitpunkt $n$ bestimmt. Die aktuelle Temperatur $T_n$ kann insbesondere gemäß Gleichung (32) bestimmt werden. In Schritt 203 wird der Hochfrequenzverstärker 10 in Abhängigkeit von dem Temperaturverlauf eingestellt.

**Patentansprüche**

1.  Verfahren zur Steuerung eines Hochfrequenzverstärkers einer Magnetresonanzanlage, umfassend:

    - Bestimmen einer von dem Hochfrequenzverstärker (10) abzugebenden Hochfrequenzleistung während einer Untersuchung eines Patienten (4) in der Magnetresonanzanlage (1),
    - Bestimmen einer Maximaltemperatur im Gewebe des Patienten (4) in Abhängigkeit von der Hochfrequenzleistung unter Verwendung einer Lambertschen Funktion, und
    - Einstellen des Hochfrequenzverstärkers (10) in Abhängigkeit von der Maximaltemperatur.

2.  Verfahren nach Anspruch 1,
    wobei die Maximaltemperatur eine lokale Maximaltemperatur in einem Gewebeabschnitt des Patienten (4) ist, wobei das Bestimmen der von dem Hochfrequenzverstärker (10) abzugebenden Hochfrequenzleistung ein Bestimmen einer von dem Hochfrequenzverstärker (10) abzugebenden lokalen Hochfrequenzleistung in dem Gewebeabschnitt während der Untersuchung des Patienten (4) in der Magnetresonanzanlage (1) umfasst.

3.  Verfahren nach Anspruch 2,
    wobei die lokale Maximaltemperatur ferner in Abhängigkeit von mindestens einem Parameter aus einer Gruppe von Parametern bestimmt wird, wobei die Gruppe von Parametern umfasst:

    - einen lokalen Parameter für eine Gefäßerweiterung,
    - eine Masse des Gewebeabschnitts, und
    - einen thermischen Leitwert aufgrund einer Perfusion in dem Gewebeabschnitt.

4.  Verfahren nach Anspruch 3,
    wobei die lokale Maximaltemperatur $T_{max}$ gemäß der Formel:

$$T_{max} = \frac{\Delta B}{\log(2)} \cdot W_0 \left( \frac{S\, m \log(2)}{G_{Perf0}\, \Delta B} \right)$$

bestimmt wird mit:

- der lokalen Hochfrequenzleistung *S* in dem Gewebeabschnitt,
- dem lokalen Parameter *ΔB* für die Gefäßerweiterung in dem Gewebeabschnitt,
- der Masse *m* des Gewebeabschnitts,
- dem thermischen Leitwert $G_{Perf0}$ aufgrund der Perfusion in dem Gewebeabschnitt, und
- der Lambertschen Funktion $W_0$.

**5.** Verfahren zur Steuerung eines Hochfrequenzverstärkers einer Magnetresonanzanlage, umfassend:

- Bestimmen einer von dem Hochfrequenzverstärker (10) abzugebenden Hochfrequenzleistung während einer Untersuchung eines Patienten (4) in der Magnetresonanzanlage (1),
- Bestimmen eines zeitlichen Temperaturverlaufs (60, 70) im Gewebe des Patienten (4) umfassend zumindest eine vorhergehende Temperatur in dem Gewebe zu einem vorhergehenden Zeitpunkt und eine aktuelle Temperatur in dem Gewebe zu einem aktuellen Zeitpunkt, wobei die aktuelle Temperatur in Abhängigkeit von der vorhergehenden Temperatur und in Abhängigkeit von der Hochfrequenzleistung zu dem aktuellen Zeitpunkt bestimmt wird, und
- Einstellen des Hochfrequenzverstärkers (10) in Abhängigkeit von dem Temperaturverlauf.

**6.** Verfahren nach Anspruch 5,
wobei der Temperaturverlauf ein lokaler Temperaturverlauf in einem Gewebeabschnitt des Patienten ist, wobei das Bestimmen der von dem Hochfrequenzverstärker (10) abzugebenden Hochfrequenzleistung ein Bestimmen einer von dem Hochfrequenzverstärker abzugebenden lokalen Hochfrequenzleistung in dem Gewebeabschnitt während der Untersuchung des Patienten (4) in der Magnetresonanzanlage (1) umfasst.

**7.** Verfahren nach Anspruch 6,
wobei der lokale Temperaturverlauf ferner in Abhängigkeit von mindestens einem Parameter aus einer Gruppe von Parametern bestimmt wird, wobei die Gruppe von Parametern umfasst:

- einen lokalen Parameter für die Gefäßerweiterung in dem Gewebeabschnitt,
- eine Masse des Gewebeabschnitts,
- einen thermischen Leitwert aufgrund der Perfusion in dem Gewebeabschnitt,
- eine Zeitdifferenz zwischen dem aktuellen Zeitpunkt und dem vorhergehenden Zeitpunkt, und
- eine Wärmekapazität des Gewebeabschnitts.

**8.** Verfahren nach Anspruch 7,
wobei die aktuelle Temperatur $T_n$ zum dem aktuellen Zeitpunkt gemäß der Formel:

$$T_n = T_{n-1} + \frac{\Delta t \cdot G_{Perf0}}{C} \cdot \left( \frac{S\, m}{G_{Perf0}\, \Delta B} - 2^{\frac{T_{n-1}}{\Delta B}} T_{n-1} \right)$$

bestimmt wird mit:

- der lokalen Hochfrequenzleistung S in dem Gewebeabschnitt,
- dem lokalen Parameter *ΔB* für die Gefäßerweiterung in dem Gewebeabschnitt,
- der Masse *m* des Gewebeabschnitts,
- dem thermischen Leitwert $G_{Perf0}$ aufgrund der Perfusion in dem Gewebeabschnitt,
- der vorhergehende Temperatur $T_{n-1}$ zum dem vorhergehenden Zeitpunkt
- der Zeitdifferenz *Δt* zwischen dem aktuellen Zeitpunkt und dem vorhergehenden Zeitpunkt, und
- der Wärmekapazität C des Gewebeabschnitts.

9. Magnetresonanzanlage umfassend:

   - ein Gradientenfeldsystem,
   - eine Hochfrequenzantenne,
   - einen Hochfrequenzverstärker (10), welcher mit der Hochfrequenzantenne zum Ausgeben eines Hochfrequenzsignals gekoppelt ist, und
   - eine Steuereinrichtung (6) zur Ansteuerung des Gradientenfeldsystems und des Hochfrequenzverstärkers (10), zum Empfang der von der Hochfrequenzantenne aufgenommenen Messsignale, zur Auswertung der Messsignale und zur Erstellung von Magnetresonanzdaten,

   wobei die Magnetresonanzanlage (1) ausgestaltet ist,
   eine von einem Hochfrequenzverstärker (10) der Magnetresonanzanlage (1) abzugebende Hochfrequenzleistung während einer Untersuchung eines Patienten (4) in der Magnetresonanzanlage zu bestimmen,
   eine Maximaltemperatur im Gewebe des Patienten (4) in Abhängigkeit von der Hochfrequenzleistung unter Verwendung einer Lambertschen Funktion zu bestimmen, und
   den Hochfrequenzverstärker (10) in Abhängigkeit von der Maximaltemperatur einzustellen.

10. Magnetresonanzanlage nach Anspruch 9, wobei die Magnetresonanzanlage (1) zur Durchführung des Verfahrens nach einem der Ansprüche 1-8 ausgestaltet ist.

11. Magnetresonanzanlage umfassend:

   - ein Gradientenfeldsystem,
   - eine Hochfrequenzantenne,
   - einen Hochfrequenzverstärker (10), welcher mit der Hochfrequenzantenne zum Ausgeben eines Hochfrequenzsignals gekoppelt ist, und
   - eine Steuereinrichtung (6) zur Ansteuerung des Gradientenfeldsystems und des Hochfrequenzverstärkers (10), zum Empfang der von der Hochfrequenzantenne aufgenommenen Messsignale, zur Auswertung der Messsignale und zur Erstellung von Magnetresonanzdaten,

   wobei die Magnetresonanzanlage (1) ausgestaltet ist,

   eine von einem Hochfrequenzverstärker (10) der Magnetresonanzanlage (1) abzugebende Hochfrequenzleistung während einer Untersuchung eines Patienten (4) in der Magnetresonanzanlage (1) zu bestimmen,
   einen zeitlichen Temperaturverlauf im Gewebe des Patienten (4) zu bestimmen, welcher zumindest eine vorhergehende Temperatur in dem Gewebe zu einem vorhergehenden Zeitpunkt und eine aktuelle Temperatur in dem Gewebe zu einem aktuellen Zeitpunkt umfasst, wobei die aktuelle Temperatur in Abhängigkeit von der vorhergehenden Temperatur und in Abhängigkeit von der Hochfrequenzleistung zu dem aktuellen Zeitpunkt bestimmt wird, und
   den Hochfrequenzverstärker (10) in Abhängigkeit von dem Temperaturverlauf einzustellen.

12. Magnetresonanzanlage nach Anspruch 11, wobei die Magnetresonanzanlage (1) zur Durchführung des Verfahrens nach einem der Ansprüche 1-8 ausgestaltet ist.

13. Computerprogrammprodukt, welches ein Programm umfasst und direkt in einen Speicher einer programmierbaren Steuereinrichtung (6) einer Magnetresonanzanlage (1) ladbar ist, mit Programmmitteln, um alle Schritte des Verfahrens nach einem oder mehreren der Ansprüche 1-8 auszuführen, wenn das Programm in der Steuereinrichtung (6) der Magnetresonanzanlage (1) ausgeführt wird.

14. Elektronisch lesbarer Datenträger mit darauf gespeicherten elektronisch lesbaren Steuerinformationen, welche derart ausgestaltet sind, dass sie bei Verwendung des Datenträgers (9) in einer Steuereinrichtung (6) einer Magnetresonanzanlage (1) das Verfahren nach einem oder mehreren der Ansprüche 1-8 durchführen.

**Geänderte Patentansprüche gemäss Regel 137(2) EPÜ.**

1. Verfahren zur Steuerung eines Hochfrequenzverstärkers einer Magnetresonanzanlage, umfassend:

- Bestimmen einer von dem Hochfrequenzverstärker (10) abzugebenden Hochfrequenzleistung während einer Untersuchung eines Patienten (4) in der Magnetresonanzanlage (1),
- Bestimmen einer Maximaltemperatur im Gewebe des Patienten (4) in Abhängigkeit von der Hochfrequenzleistung unter Verwendung einer Lambertschen Funktion, und
- Einstellen des Hochfrequenzverstärkers (10) in Abhängigkeit von der Maximaltemperatur,

wobei die Maximaltemperatur eine lokale Maximaltemperatur in einem Gewebeabschnitt des Patienten (4) ist, wobei das Bestimmen der von dem Hochfrequenzverstärker (10) abzugebenden Hochfrequenzleistung ein Bestimmen einer von dem Hochfrequenzverstärker (10) abzugebenden lokalen Hochfrequenzleistung in dem Gewebeabschnitt während der Untersuchung des Patienten (4) in der Magnetresonanzanlage (1) umfasst, wobei die lokale Maximaltemperatur ferner in Abhängigkeit von

- einem lokalen Parameter für eine Gefäßerweiterung,
- einer Masse des Gewebeabschnitts, und
- einem thermischen Leitwert aufgrund einer Perfusion in dem Gewebeabschnitt unter Verwendung der Lambertschen Funktion bestimmt wird.

2. Verfahren nach Anspruch 1, wobei die lokale Maximaltemperatur $T_{max}$ gemäß der Formel:

$$T_{max} = \frac{\Delta B}{\log(2)} \cdot W_0\left(\frac{S\, m\, \log(2)}{G_{Perf0}\, \Delta B}\right)$$

bestimmt wird mit:

- der lokalen Hochfrequenzleistung S in dem Gewebeabschnitt,
- dem lokalen Parameter $\Delta B$ für die Gefäßerweiterung in dem Gewebeabschnitt,
- der Masse m des Gewebeabschnitts,
- dem thermischen Leitwert $G_{Perf0}$ aufgrund der Perfusion in dem Gewebeabschnitt, und
- der Lambertschen Funktion $W_0$.

3. Verfahren zur Steuerung eines Hochfrequenzverstärkers einer Magnetresonanzanlage, umfassend:

- Bestimmen einer von dem Hochfrequenzverstärker (10) abzugebenden Hochfrequenzleistung während einer Untersuchung eines Patienten (4) in der Magnetresonanzanlage (1),
- Bestimmen eines zeitlichen Temperaturverlaufs (60, 70) im Gewebe des Patienten (4) umfassend zumindest eine vorhergehende Temperatur in dem Gewebe zu einem vorhergehenden Zeitpunkt und eine aktuelle Temperatur in dem Gewebe zu einem aktuellen Zeitpunkt, wobei die aktuelle Temperatur in Abhängigkeit von der vorhergehenden Temperatur und in Abhängigkeit von der Hochfrequenzleistung zu dem aktuellen Zeitpunkt bestimmt wird, und
- Einstellen des Hochfrequenzverstärkers (10) in Abhängigkeit von dem Temperaturverlauf,

wobei der Temperaturverlauf ein lokaler Temperaturverlauf in einem Gewebeabschnitt des Patienten ist, wobei das Bestimmen der von dem Hochfrequenzverstärker (10) abzugebenden Hochfrequenzleistung ein Bestimmen einer von dem Hochfrequenzverstärker abzugebenden lokalen Hochfrequenzleistung in dem Gewebeabschnitt während der Untersuchung des Patienten (4) in der Magnetresonanzanlage (1) umfasst, **dadurch gekennzeichnet, dass** die aktuelle Temperatur $T_n$ zum dem aktuellen Zeitpunkt gemäß der Formel:

$$T_n = T_{n-1} + \frac{\Delta t \cdot G_{Perf0}}{C} \cdot \left(\frac{S\, m}{G_{Perf0}\, \Delta B} - 2^{\frac{T_{n-1}}{\Delta B}} T_{n-1}\right)$$

bestimmt wird mit:

- der lokalen Hochfrequenzleistung S in dem Gewebeabschnitt,
- einem lokalen Parameter $\Delta B$ für die Gefäßerweiterung in dem Gewebeabschnitt,

- einer Masse $m$ des Gewebeabschnitts,
- einem thermischen Leitwert $G_{Perf0}$ aufgrund der Perfusion in dem Gewebeabschnitt,
- der vorhergehenden Temperatur $T_{n-1}$ zum dem vorhergehenden Zeitpunkt
- einer Zeitdifferenz $\Delta t$ zwischen dem aktuellen Zeitpunkt und dem vorhergehenden Zeitpunkt, und
- einer Wärmekapazität C des Gewebeabschnitts.

4.  Magnetresonanzanlage umfassend:

   - ein Gradientenfeldsystem,
   - eine Hochfrequenzantenne,
   - einen Hochfrequenzverstärker (10), welcher mit der Hochfrequenzantenne zum Ausgeben eines Hochfrequenzsignals gekoppelt ist, und
   - eine Steuereinrichtung (6) zur Ansteuerung des Gradientenfeldsystems und des Hochfrequenzverstärkers (10), zum Empfang der von der Hochfrequenzantenne aufgenommenen Messsignale, zur Auswertung der Messsignale und zur Erstellung von Magnetresonanzdaten,

   wobei die Magnetresonanzanlage (1) zur Durchführung des Verfahrens nach einem der Ansprüche 1-3 ausgestaltet ist.

5.  Computerprogrammprodukt, welches ein Programm umfasst und direkt in einen Speicher einer programmierbaren Steuereinrichtung (6) einer Magnetresonanzanlage (1) ladbar ist, mit Programmmitteln, um alle Schritte des Verfahrens nach einem oder mehreren der Ansprüche 1-3 auszuführen, wenn das Programm in der Steuereinrichtung (6) der Magnetresonanzanlage (1) ausgeführt wird.

6.  Elektronisch lesbarer Datenträger mit darauf gespeicherten elektronisch lesbaren Steuerinformationen, welche derart ausgestaltet sind, dass sie bei Verwendung des Datenträgers (9) in einer Steuereinrichtung (6) einer Magnetresonanzanlage (1) das Verfahren nach einem oder mehreren der Ansprüche 1-3 durchführen.

FIG 1

FIG 2

FIG 3

FIG 4

FIG 5

FIG 6

FIG 7

FIG 8

FIG 9

FIG 10

FIG 11

## FIG 12

## FIG 13

## FIG 14

**Europäisches Patentamt**
**European Patent Office**
**Office européen des brevets**

## EUROPÄISCHER RECHERCHENBERICHT

Nummer der Anmeldung

EP 18 19 9924

### EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | ESRA NEUFELD ET AL: "Rapid method for thermal dose-based safety supervision during MR scans : Rapid Method for Thermal Dose-Based Safety Supervision", BIOELECTROMAGNETICS, Bd. 36, Nr. 5, 1. Mai 2015 (2015-05-01), Seiten 398-407, XP055585068, US ISSN: 0197-8462, DOI: 10.1002/bem.21919 | 5-7, 11-14 | INV. G01R33/28 |
| A | * Chapters Introduction, Methods: background, thermal dose model, temperature increase, algorithm; Abbildungen 4, 8 * | 1-4,8-10 | |
| X | US 2017/307710 A1 (BOULANT NICOLAS [FR] ET AL) 26. Oktober 2017 (2017-10-26) | 5-7, 11-14 | |
| A | * Absätze [0004], [0007], [0008], [0032] - [0061], [0065], [0066]; Ansprüche 1,6; Abbildungen 1,2, 4,7 * | 1-4 | |
| A | GHOSH SUVRADIP ET AL: "A Circuit for Energy Harvesting Using On-Chip Solar Cells", IEEE TRANSACTIONS ON POWER ELECTRONICS, INSTITUTE OF ELECTRICAL AND ELECTRONICS ENGINEERS, USA, Bd. 29, Nr. 9, 1. September 2014 (2014-09-01), Seiten 4658-4671, XP011546722, ISSN: 0885-8993, DOI: 10.1109/TPEL.2013.2273674 [gefunden am 2014-04-30] * page 4661-4663: chapter IV. circuit analysis A.-C.; Abbildungen 11,12 * | 1-4,9, 10,13,14 | RECHERCHIERTE SACHGEBIETE (IPC) G01R |

Der vorliegende Recherchenbericht wurde für alle Patentansprüche erstellt

| Recherchenort | Abschlußdatum der Recherche | Prüfer |
|---|---|---|
| München | 9. Mai 2019 | Faber-Jurk, Sonja |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

EPO FORM 1503 03.82 (P04C03)

**ANHANG ZUM EUROPÄISCHEN RECHERCHENBERICHT
ÜBER DIE EUROPÄISCHE PATENTANMELDUNG NR.**

EP 18 19 9924

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

09-05-2019

| Im Recherchenbericht angeführtes Patentdokument | Datum der Veröffentlichung | Mitglied(er) der Patentfamilie | Datum der Veröffentlichung |
|---|---|---|---|
| US 2017307710    A1 | 26-10-2017 | US    2017307710 A1<br>WO    2016049013 A1 | 26-10-2017<br>31-03-2016 |

EPO FORM P0461

Für nähere Einzelheiten zu diesem Anhang : siehe Amtsblatt des Europäischen Patentamts, Nr.12/82

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 6762605 B2
- US 9625541 B2
- US 10031193 B2
- US 9989603 B2
- US 8547097 B2
- US 9547053 B2

**In der Beschreibung aufgeführte Nicht-Patentliteratur**

- **M. MURBACH et al.** Virtual Population-Based Assessment of the Impact of 3 Tesla Radiofrequency Shimming and Thermoregulation on Safety and B1+ Uniformity. *Magnetic Resonance in Medicine,* 2016, vol. 76, 986-997
- **E. NEUFELD et al.** Rapid method for thermal dose-based safety supervision during MR scans. *Biomagnetics*
- **H. H. PENNES.** Analysis of tissue and arterial blood temperatures in the resting human forearm. *Journal of Applied Physiology,* 1948, vol. 1, 93-122